# EUROPEAN PATENT APPLICATION

(11) **EP 4 414 448 A1**
(43) Date of publication of application: **14.08.2024**
(21) Application number: 22912896.2
(22) Date of filing: 24.01.2022
(51) Int. Cl.: C12N 9/10, C12N 15/54, C12N 1/21, C12N 1/19, C12P 17/04, C12P 13/00, C12P 17/00

(54) **TRANSAMINASE MUTANT AND USE THEREOF**

(30) Priority: 29.12.2021 CN 202111645514
(71) Applicant: Asymchem Laboratories (Tianjin) Co., Ltd., Tianjin 300457 (CN)
(72) Inventor: HONG, Hao, Morrisville, NC 27560 (US); JAMES, Gage, Morrisville, NC 27560 (US); ZHANG, Na, Tianjin 300457 (CN); JIAO, Xuecheng, Tianjin 300457 (CN); LI, Rui, Tianjin 300457 (CN); YANG, Yiming, Tianjin 300457 (CN); ZHANG, Yanqing, Tianjin 300457 (CN)
(74) Representative: Berggren Oy
(86) International application number: PCT/CN2022/073561
(87) International publication number: WO 2023/123589

(57) **Abstract**

Disclosed are a transaminase mutant and the use thereof, herein the transaminase mutant has a sequence formed after an amino acid mutation occurs in a sequence as shown in SEQ ID NO: 1, and the site at which the amino acid mutation occurs includes a V242W site. On the basis of transaminase shown in SEQ ID NO: 1, the transaminase mutant undergoes the mutation by means of a site-directed mutation method, thereby the amino acid sequence of the transaminase is changed and changes in protein structure and function are achieved, and then the transaminase with the above mutation site is obtained by means of a directed screening method. The transaminase obtained has relatively high catalytic activity, specific selectivity, wide substrate spectrum and broad industrial prospect.

## Description

### Technical Field

The present disclosure relates to the field of biotechnologies, in particular to a transaminase mutant and use thereof.

### Background

Chiral amine compounds are an important chiral pharmaceutical intermediate, which is extensively applied in fields such as medicines. Industrial production mainly relies on asymmetric synthesis using transition metal catalysts, but this process requires expensive transition metal complexes as a catalyst, and due to the limited resources and high costs of these transition metals, this way is difficult to achieve sustainability. A bio-enzyme catalysis method attracts much attention due to the wide range of substrate ketones, mild reaction conditions, and high product selectivity.

Transaminase (TA, EC2.6.1.X), also known as aminotransferase, may reversibly catalyze an amino transfer reaction between ketone groups and amino groups. Herein, the transaminase has excellent stereoselectivity, renewable cofactors, strong reaction activity, and environmental friendliness, and it is used for biocatalytic production of chiral amines which are already widely used in the synthesis of pharmaceutical and pesticide intermediates.

The transaminase reaction usually requires pyridoxal phosphate as a coenzyme, the pyridoxal phosphate covalently binds to an ε-amino of a lysine residue at the active center of the transaminase, and an amino donor is also required to participate in the reaction, commonly used amino donors include isopropylamine, phenylethylamine and the like.

Although the progress in the production of the chiral amines using the transaminase already receives high attention, there are still many problems for enzymatic methods in enlarge production applications, such as poor selectivity of existing transaminases and reversible reactions that may lead to low conversion rates, it is not beneficial to industrial production.

### Summary

The present disclosure aims to provide a transaminase mutant and use thereof, as to improve the selectivity of transaminases.

In order to achieve the above purpose, according to one aspect of the present disclosure, a transaminase mutant is provided. The transaminase mutant has an amino acid sequence obtained by the mutation of the amino acid sequence shown in SEQ ID NO: 1, and the mutation includes V242W, or an amino acid sequence has the mutation and has more than 80%, preferably more than 90%, and more preferably more than 95% of the identity with SEQ ID NO: 1; alternatively, the amino acid sequence of the transaminase mutant has the mutation, has more than 90% of the homology with the sequence as shown in SEQ ID NO: 1, and has the transaminase catalytic activity.

Further, the mutation includes any one of the following mutation site combinations: V242W+L59Q, V242W+F164C, V242W+F164Q, V242W+F164W, V242W+F164Y, V242W+L272G, V242W+L272I, V242W+L272K, V242W+L272M, V242W+L272P, V242W+L272V, V242W+L272Y, V242W+V328C, V242W+V328I, V242W+V328L, V242W+V328M, V242W+V328Q, V242W+V328S, V242W+V328T, V242W+V328W, V242W+T330F, V242W+T330I, V242W+T330S, V242W+A436H, V242W+A436K, V242W+A436L, V242W+A436N, V242W+A436P, V242W+A436Q, V242W+A436S, V242W+A436Y, V242W+R442A, V242W+R442C, V242W+R442F, V242W+R442G, V242W+R442H, V242W+R442N, V242W+R442Q, V242W+R442S, V242W+R442T, V242W+F164Q+V328A, V242W+F164Q+V328C, V242W+F164Q+V328D, V242W+F164Q+V328E, V242W+F164Q+V328F, V242W+F164Q+V328G, V242W+F164Q+V328H, V242W+F164Q+V328I, V242W+F164Q+V328L, V242W+F164Q+V328M, V242W+F164Q+V328P, V242W+F164Q+V328Q, V242W+F164Q+V328R, V242W+F164Q+V328S, V242W+F164Q+V328W, V242W+F164Q+V328T, V242W+F164Q+V328Y, V242W+F164Q+R442T, V242W+F164Q+V328I+G2S, V242W+F164Q+V328I+T46M, V242W+F164Q+V328I+G48D, V242W+F164Q+V328I+C185Y, V242W+F164Q+V328I+S186N, V242W+F164Q+V328I+S194P, V242W+F164Q+V328I+T197M, V242W+F164Q+V328I+N202D, V242W+F164Q+V328I+Y205L , V242W+F164Q+V328I+T245A, V242W+F164Q+V328I+V252I, V242W+F164Q+V328I+S268N, V242W+F164Q+V328I+L353F, V242W+F164Q+V328I+N359D, V242W+F164Q+V328I+R409T, V242W+F164Q+V328I+E424K, V242W+F164Q+V328I+A436V, V242W+F164Q+V328I+R442T, V242W+F164Q+V328I+R442T+G48D, V242W+F164Q+V328I+R442T+S194P, V242W+F164Q+V328I+R442T+V252I, V242W+F164Q+V328I+R442T+S194P+V252I, V242W+F164Q+V328I+R442T+V252I+G48D, V242W+F164Q+V328I+R442T+S194P+G48D or V242W+F164Q+V328I+R442T+S194P+V252I+G48D, or the amino acid sequence of the transaminase mutant includes the above mutation site combination and has more than 80%, preferably more than 90%, and more preferably more than 95% of the identity with SEQ ID NO: 1.

According to another aspect of the present disclosure, a DNA molecule is provided. The DNA molecule encodes any one of the above transaminase mutants.

According to another aspect of the present disclosure, a recombinant plasmid is provided. The recombinant plasmid contains any one of the above DNA molecules.

Further, the recombinant plasmid is pET-22a (+), pET-22b (+), pET-3a (+), pET-3d (+), pET-11a (+), pET-12a (+), pET-14b (+), pET-15b (+), pET-16b (+), pET-17b (+), pET-19b (+), pET-20b (+), pET-21a (+), pET-23a (+), pET-23b (+), pET-24a (+), pET-25b (+), pET-26b (+), pET-27b (+), pET-28a (+), pET-29a (+), pET-30a (+), pET-31b (+), pET-32a (+), pET-35b (+), pET-38b (+), pET-39b (+), pET-40b (+), pET-41a (+), pET-41b (+), pET-42a (+), pET-43a (+), pET-43b (+), pET-44a (+), pET-49b (+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-18, pUC-18 or pUC-19.

According to another aspect of the present disclosure, a host cell is provided. The host cell contains any one of the above recombinant plasmids.

Further, the host cell includes a prokaryotic cell or a eukaryotic cell; preferably the prokaryotic cell is a BL21-DE3 cell or an *Escherichia coli* Rosetta-DE3 cell; and the eukaryotic cell is a yeast cell.

According to another aspect of the present disclosure, a method for producing a chiral amine is provided. The method includes a step of performing a catalytic transamination reaction on a ketone compound and an amino donor using a transaminase, and the transaminase is any one of the above transaminase mutants.

Further, the ketone compound is 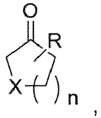 n=0, 1, 2 or 3; X=C, N, O or S; R=H, F, Cl, Br, CH₃ or CH₂CH₃; and preferably the ketone compound is 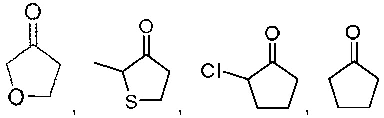 or

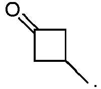

Further, the amino donor is isopropamide or alanine, preferably the isopropamide.

Further, in a reaction system for performing the catalytic transamination reaction on the ketone compound and the amino donor using the transaminase, the amount of enzyme used is 1.5-6.6 mg/mL, preferably 1.7 mg/mL; and preferably, in the reaction system for performing the catalytic transamination reaction on the ketone compound and the amino donor using the transaminase, the temperature is 20 °C ~45 °C, more preferably 30 °C.

On the basis of the transaminase shown in SEQ ID NO: 1, the above transaminase mutant of the present disclosure undergoes the mutation by means of a site-directed mutation method, thereby its amino acid sequence is changed and changes in protein structure and function are achieved, and then the transaminase with the above mutation site is obtained by means of a directed screening method. The transaminase obtained in the present disclosure has relatively high catalytic activity, specific selectivity, wide substrate spectrum and broad industrial prospect.

### Detailed Description of the Embodiments

It should be noted that, in the case without conflicting, embodiments in the present application and features in the embodiments may be combined with each other. The present disclosure is described in detail below in combination with the embodiments.

Transaminases are a class of biocatalysts primarily composed of proteins, and a reaction catalyzed by it may be expressed by the following reaction formula: 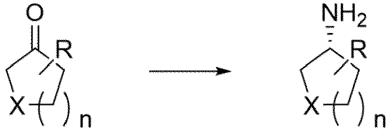 n=0, 1, 2, or 3; X=C, N, O or S; R=H, F, Cl, Br, CH₃ or CH₂CH₃

The existing transaminases have poor selectivity and reversible reactions that may lead to low conversion rates, it is not beneficial to industrial production. In response to this technical problem, the present disclosure improves the selectivity and activity of the transaminases by a site-directed evolution method based on the transaminase as shown in SEQ ID NO: 1, to obtain a transaminase with relatively high catalytic activity and specific selectivity.

Firstly, a mutation site is introduced on the transaminase by the site-directed mutagenesis mode, to selectively detect mutants and select the mutant with improved selectivity. Herein, the mutant V242W has a selectivity increase of about 2 times compared to a starting template, but its activity is poor. Subsequently, mutations are continuously performed by using V242W as a template in order to obtain the mutant with significantly improved selectivity and activity.

Site-directed mutagenesis: refers to introduction of base changes or insertion and deletion of fragments into a specific site of a DNA fragment (may be a genome, or a plasmid) by methods such as a polymerase chain reaction (PCR). The site-directed mutagenesis may rapidly and efficiently improve the characters and representation of a target protein expressed by DNA, and is a very useful means in genetic research work.

The introduction of single or multiple site-specific mutations by using the whole plasmid PCR has the advantages of simplicity and efficiency and the like. A principle thereof is: a pair of primers (forward and reverse) containing the mutation sites, and a template plasmid are annealed to "cyclic extension" with a polymerase, the so-called cyclic extension is that the polymerase extends the primer according to the template, it is returned and terminated at a 5'-end of the primer after a circle, and subjected to the cycle of repeated heating, annealing and extending. This reaction is different from rolling circle amplification, and may not form a plurality of tandem copies. After annealing, extension products of the forward and reverse primers are paired to become a nicked open-circle plasmid. A Dpn I enzyme-digested extension product, because the original template plasmid is derived from conventional *Escherichia coli,* is modified by dam methylation, is sensitive to Dpn I and is cut up, while the plasmid with a mutated sequence synthesized in vitro is not cut up because there is no methylation, and therefore it is successfully transformed in the subsequent transformation, to obtain a clone of a mutant plasmid.

The mutant plasmid is transformed into the host cell of *Escherichia coli,* and then a crude enzyme obtained by a method of cell ultrasonication is used for reaction validation. The optimal conditions for inducing transaminase expression are: 20 °C, and 0.06 mM isopropyl-β-d-thiogalactoside (IPTG) induction for 16 h.

According to a typical implementation of the present disclosure, a transaminase mutant is provided. The transaminase mutant has an amino acid sequence obtained by the mutation of the amino acid sequence shown in SEQ ID NO: 1, and the mutation comprises V242W, or an amino acid sequence has the mutation and has more than 90% homology with SEQ ID NO: 1and has transaminase activity.

The term "homology" used in this disclosure has a commonly known meaning in this field, and those skilled in the art are also familiar with the rules and standards for determining the homology between different sequences. Those skilled in the art may obtain such variant sequences under the guidance of the disclosed content of the present application.

Preferably, the mutation includes any one of the following mutation site combinations: V242W+L59Q, V242W+F164C, V242W+F164Q, V242W+F164W, V242W+F164Y, V242W+L272G, V242W+L272I, V242W+L272K, V242W+L272M, V242W+L272P, V242W+L272V, V242W+L272Y, V242W+V328C, V242W+V328I, V242W+V328L, V242W+V328M, V242W+V328Q, V242W+V328S, V242W+V328T, V242W+V328W, V242W+T330F, V242W+T330I, V242W+T330S, V242W+A436H, V242W+A436K, V242W+A436L, V242W+A436N, V242W+A436P, V242W+A436Q, V242W+A436S, V242W+A436Y, V242W+R442A, V242W+R442C, V242W+R442F, V242W+R442G, V242W+R442H, V242W+R442N, V242W+R442Q, V242W+R442S, V242W+R442T, V242W+F164Q+V328A, V242W+F164Q+V328C, V242W+F164Q+V328D, V242W+F164Q+V328E, V242W+F164Q+V328F, V242W+F164Q+V328G, V242W+F164Q+V328H, V242W+F164Q+V328I, V242W+F164Q+V328L, V242W+F164Q+V328M, V242W+F164Q+V328P, V242W+F164Q+V328Q, V242W+F164Q+V328R, V242W+F164Q+V328S, V242W+F164Q+V328W, V242W+F164Q+V328T, V242W+F164Q+V328Y, V242W+F164Q+R442T, V242W+F164Q+V328I+G2S, V242W+F164Q+V328I+T46M, V242W+F164Q+V328I+G48D, V242W+F164Q+V328I+C185Y, V242W+F164Q+V328I+S186N, V242W+F164Q+V328I+S194P, V242W+F164Q+V328I+T197M, V242W+F164Q+V328I+N202D, V242W+F164Q+V328I+Y205L , V242W+F164Q+V328I+T245A, V242W+F164Q+V328I+V252I, V242W+F164Q+V328I+S268N, V242W+F164Q+V328I+L353F, V242W+F164Q+V328I+N359D, V242W+F164Q+V328I+R409T, V242W+F164Q+V328I+E424K, V242W+F164Q+V328I+A436V, V242W+F164Q+V328I+R442T, V242W+F164Q+V328I+R442T+G48D, V242W+F164Q+V328I+R442T+S194P, V242W+F164Q+V328I+R442T+V252I, V242W+F164Q+V328I+R442T+S194P+V252I, V242W+F164Q+V328I+R442T+V252I+G48D, V242W+F164Q+V328I+R442T+S194P+G48D or V242W+F164Q+V328I+R442T+S194P+V252I+G48D.

On the basis of the transaminase shown in SEQ ID NO: 1, the above transaminase mutant of the present disclosure undergoes the mutation by means of a site-directed mutation method, thereby its amino acid sequence is changed and changes in protein structure and function are achieved, and then the transaminase with the above mutation site is obtained by means of a directed screening method. The transaminase obtained in the present disclosure has relatively high catalytic activity, specific selectivity, wide substrate spectrum and broad industrial prospect.

According to a typical implementation of the present disclosure, a DNA molecule is provided. The transaminase mutant encoded by the above DNA has improved selectivity and activity, which may reduce the amount of enzyme added and reduce the difficulty of post-treatment in the industrial production of amino acids.

The above DNA molecule of the disclosure may also exist in the form of an "expression cassette". The "expression cassette" refers to a linear or circular nucleic acid molecule that encompasses DNA and RNA sequences capable of guiding expression of a specific nucleotide sequence in an appropriate host cell. Generally, including a promoter which is effectively linked with a target nucleotide, it is optionally effectively linked with a termination signal and/or other control elements. The expression cassette may also include a sequence required for proper translation of the nucleotide sequence. A coding region usually encodes a target protein, but also encodes a target function RNA in a sense or antisense direction, for example an antisense RNA or an untranslated RNA. The expression cassette including a target polynucleotide sequence may be chimeric, which means that at least one of components thereof is heterologous to at least one of the other components thereof. The expression cassette may also be existent naturally, but obtained with effective recombinant formation for heterologous expression.

According to a typical implementation of the disclosure, a recombinant plasmid is provided. The recombinant plasmid contains any one of the above DNA molecules. The DNA molecule in the above recombinant plasmid is placed in a proper position of the recombinant plasmid, so that the above DNA molecule may be correctly and smoothly copied, transcribed or expressed.

Although a qualifier used in the disclosure while the above DNA molecule is defined is "contain", it does not mean that other sequences which are not related to a function thereof may be arbitrarily added to both ends of the DNA sequence. Those skilled in the art know that in order to meet the requirements of recombination operations, it is necessary to add suitable enzyme digestion sites of a restriction enzyme at two ends of the DNA sequence, or additionally increase a start codon, a termination codon and the like, therefore, if the closed expression is used for defining, these situations may not be covered truly.
typical implementationThe term "plasmid" used in the disclosure includes any plasmids, cosmids, bacteriophages or agrobacterium binary nucleic acid molecules in double-strand or single-strand linear or circular form, preferably a recombinant expression plasmid, which may be a prokaryotic expression plasmid or may be a eukaryotic expression plasmid, preferably the prokaryotic expression plasmid, in some implementation, a vector used for the recombinant plasmid is selected from pET-22a (+), pET-22b (+), pET-3a (+), pET-3d (+), pET-11a (+), pET-12a (+), pET-14b (+), pET-15b (+), pET-16b (+), pET-17b (+), pET-19b (+), pET-20b (+), pET-21a (+), pET-23a (+), pET-23b (+), pET-24a (+), pET-25b (+), pET-26b (+), pET-27b (+), pET-28a (+), pET-29a (+), pET-30a (+), pET-31b (+), pET-32a (+), pET-35b (+), pET-38b (+), pET-39b (+), pET-40b (+), pET-41a (+), pET-41b (+), pET-42a (+), pET-43a (+), pET-43b (+), pET-44a (+), pET-49b (+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-18, pUC-18 or pUC-19.

According to a typical implementation of the present disclosure, a host cell is provided, and the host cell contains any one of the above recombinant plasmids. The host cell applicable to the present disclosure includes but not limited to a prokaryotic cell or a eukaryotic cell. Preferably the prokaryotic cell is a BL21-DE3 cell or an *Escherichia coli* Rosetta-DE3 cell, and the eukaryotic cell is yeast.

According to a typical implementation of the present disclosure, a method for producing a chiral amine is provided. The method includes a step of performing a catalytic transamination reaction on a ketone compound and an amino donor using a transaminase, and the transaminase is the transaminase mutant of the present disclosure. Preferably, the ketone compound is 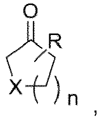 n=0, 1, 2 or 3; X=C, N, O or S; R=H, F, Cl, Br, CH₃ or CH₂CH₃.

In a reaction system for performing the catalytic transamination reaction on the ketone compound and the amino donor using the transaminase, pH is 7-11, preferably 7.5. In the reaction system for performing the catalytic transamination reaction on the ketone compound and the amino donor using the transaminase, the temperature is 20°C~45°C, more preferably 30 °C, this means that the temperature value may be arbitrarily selected as a value of 20-45 °C, such as 20, 21, 22, 25, 27, 28, 29, 20, 31, 32, 35, 37, 38, 39, 40, 42, and 45. In the reaction system for performing the catalytic transamination reaction on the ketone compound and the amino donor using the transaminase, the amount of enzyme used is 1.5-6.6 mg/mL, preferably 1.7 mg/mL, this means that the value of the enzyme amount may be arbitrarily selected as a value of 1.5-6.6 mg/mL, such as 1.5, 1.6, 1.7, 1.8, 1.9, 2.0, 2.5, 3.0, 3.6, 3.8, 4.0, 4.4, 4.5, 4.9, 5.1, 5.5, and 6.6.

In an implementation mode of the present disclosure, substrates of the transaminase mutant of the present disclosure are as follows:
Substrate 1:
   Tetrahydrofuran-3-one
Substrate 2: 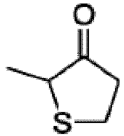 2-Methylthiolan-3-one
Substrate 3: 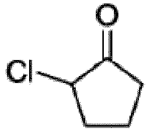 2-Chlorocyclopentanone
Substrate 4: 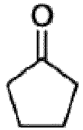 Cyclopentanone
Substrate 5: 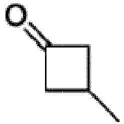 3-Methylcyclobutan-1-one

According to a typical implementation of the present disclosure, a reaction validation method for Substrate 1, Substrate 2, Substrate 3, Substrate 4, and Substrate 5 is as follows:
10 mg of a substrate, 1 mg of an enzyme, 0.1 mg of pyridoxal phosphate, and 20 mg of 6 M isopropylamine hydrochloride are respectively added to a 5 mL centrifuge tube, and 0.1 M phosphate buffer solution pH 7.5 is supplemented to a total volume of 0.5 mL, and it is reacted at 45 °C and 200 rpm for 16 h.

After the reaction is completed, a selectivity detection method for Substrates 1, 2, and 3 is as follows: 0.06 mL of a reaction system is taken, 0.04 mL of 0.1 M phosphate buffer solution pH 7.5 is added, 0.1 mL of a diluted system is taken, and 0.3 mL of acetonitrile, water, and sodium bicarbonate equal-volume solution is added, and mixed uniformly. 0.1 mL is taken, 0.9 mL of 5 mg/mL Nα-(2,4-dinitro-5-fluorophenyl)-L-alaninamide reagent is added, it is placed in a 50 °C metal bath for 3 h, a derivative system is taken and centrifuged at 12000 rpm for 5 min. 0.5 mL is taken, 0.5 mL of acetonitrile and water equal-volume solution is added, and mixed uniformly. It is sent for e.e. detection. In the present application, an activity detection method (expressed by the substrate conversion rate) is as follows: 100 µL of a reaction system after an enzyme catalyzed substrate reaction is taken, 900 µL of methanol is added, it is shaken and centrifuged to take a supernatant, and a sample is sent; and a relative peak area occupied by a product detected by a high performance liquid chromatography (HPLC) represents the activity of the enzyme.

In a typical implementation of the present disclosure, the amino donor is isopropylamine or alanine, preferably the isopropylamine.

The beneficial effects of the present disclosure are further described below in combination with embodiments.

### Embodiment 1

A site-specific mutation was performed on C60Y (Template, as shown in SEQ ID NO:1, derived from *Arthrobacter citreus*,
MGLTVQKINWEQVKEWDRKYLMRTFSTQNEYQPVPIESTEGDYLITPGGTRLLDFFNQLYCVNLG QKNQKVNAAIKEALDRYGFVWDTYATDYKAKAAKIIIEDILGDEDWPGKVRFVSTGSEAVETALNIA RLYTNRPLVVTREHDYHGWTGGAATVTRLRSFRSGLVGENSESFSAQIPGSSCSSAVLMAPSSN TFQDSNGNYLKDENGELLSVKYTRRMIENYGPEQVAAVITEVSQGVGSTMPPYEYVPQIRKMTKE LGVLWISDEVLTGFGRTGKWFGYQHYGVQPDIITMGKGLSSSSLPAGAVVVSKEIAAFMDKHRWE SVSTYAGHPVAMAAVCANLEVMMEENLVEQAKNSGEYIRSKLELLQEKHKSIGNFDGYGLLWIVDI VNAKTKTPYVKLDRNFRHGMNPNQIPTQIIMEKALEKGVLIGGAMPNTMRIGASLNVSRGDIDKAM DALDYALDYLESGEWQQSLEHHHHHH; and the corresponding nucleotide sequence is SEQ ID NO: 2, derived from *Arthrobacter citreus*, 
atgggcctgaccgtacagaagatcaactgggaacaagtaaaggagtgggaccgcaagtacctgatgcgcactttttccactcagaacg
aataccagccggtaccgattgaatctacggaaggtgattatctgatcaccccgggtggcacccgtctgctggacttcttcaaccaactgtatt
gcgttaacctgggccagaaaaaccagaaagtcaacgccgcgattaaagaggcactggaccgctacggcttcgtatgggacacttatgct
accgactacaaggcgaaagccgcgaaaatcattatcgaggatattctgggtgacgaggattggcctggtaaagttcgttttgttagcactg
gctccgaagccgtggaaaccgcgctgaatatcgcgcgtctgtataccaatcgtccgctggttgtgacccgtgaacacgactatcacggttg
gaccggcggcgctgcaaccgtcactcgtctgcgtagcttccgttctggtctggtgggtgaaaactccgaaagcttctctgctcagatcccgg
gttcttcttgtagctctgcagtcctgatggccccgtcttccaacaccttccaggactctaacggcaactacctgaaagacgaaaacggtgaa
ctgctgtccgttaaatacacccgtcgcatgattgaaaactacggcccggagcaggtagccgcagtgattacggaagttagccagggtgttg
gttctacgatgccgccatacgaatatgttccacagatccgtaaaatgactaaagagctgggcgttctgtggatctccgacgaggtcctgacc
ggttttggtcgtaccggtaaatggttcggttaccagcattatggcgttcaaccagatatcattactatgggcaagggcctgtcttcttcctctctgc
cggcgggcgcagtagttgtgtccaaagaaatcgctgcgtttatggacaaacaccgctgggaatctgtttccacctacgcgggtcacccggt
ggcgatggcagctgtgtgcgctaacctggaagtgatgatggaagaaaacctggtggaacaagctaaaaatagcggcgaatacatccgt
agcaaactggaactgctgcaggaaaagcataaaagcatcggcaacttcgacggttacggtctgctgtggattgttgatatcgtgaatgcg
 
aaaactaaaaccccgtatgtcaaactggatcgcaacttccgccacggcatgaacccgaaccagatcccgacgcagattatcatggaaa
aagctctggaaaaaggcgttctgatcggtggtgcaatgcctaacactatgcgtatcggcgcatctctgaacgtatctcgtggtgatatcgata
aagctatggatgctctggattacgcgctggattacctggagtccggtgaatggcagcagagcctcgagcaccaccaccaccaccactga
 ), the mutation was performed at L59, Y60, Y148, H149, F164, E237, V242, V271, L272, and V328, and there was a total of 10 mutation sites and 72 mutants.

Reaction validation was performed on Substrate 1, and a reaction system was as follows: 10 mg of the substrate, 0.1 mg of pyridoxal phosphate, 20 mg of 6 M isopropylamine hydrochloride, and 1 mg of an enzyme were supplemented with 0.1 M phosphate buffer solution pH 7.5 until the total volume was 0.5 mL, and reaction conditions were: 45 °C, 200 rpm, and 16 h. Herein, mutants L59Q, L59W, H149D, H149I, H149R, F164W, V242W, V242H, V242Q, and V328I showed significant improvement, herein V242W had the highest e.e., which was increased by about 2 times compared to Template, but the activity (substrate conversion rate) was decreased. Results were shown in Table 1.

**Table 1**

| Mutation site | Substrate 1 e.e.% | Substrate 1 activity% |
|---|---|---|
| Template | ++ | *** |
| L59A | + | ** |
| L59D | ++ | * |
| L59F | + | * |
| L59G | + | * |
| L59K | ++ | * |
| L59L | ++ | ** |
| L59N | - | *** |
| L59Q | +++ | ** |
| L59R | - | * |
| L59S | + | ** |
| L59V | + | ** |
| L59W | ++++ | * |
| Y60E | + | ** |
| Y60G | ++ | ** |
| Y60N | + | * |
| Y60Q | + | ** |
| Y60V | + | * |
| Y148L | + | * |
| Y148M | - | * |
| Y148Q | ++ | * |
| Y148T | - | * |
| H149D | +++ | * |
| H149F | + | * |
| H149I | ++++ | * |
| H149M | + | * |
| H149N | + | * |
| H149R | +++ | * |
| H149S | + | * |
| H149T | ++ | * |
| H149Y | + | * |
| F164P | ++ | ** |
| F164R | - | ** |
| F164W | +++ | ** |
| F164Y | + | ** |
| E237C | ++ | * |
| E237G | ++ | * |
| E237Q | +++ | * |
| E237S | + | *** |
| V242A | ++ | ** |
| V242F | + | * |
| V242H | +++ | ** |
| V242l | + | * |
| V242L | + | ** |
| V242N | + | *** |
| V242P | + | ** |
| V242Q | +++ | ** |
| V242R | + | * |
| V242S | + | ** |
| V242T | ++ | ** |
| V242W | +++++ | * |
| V242Y | ++ | * |
| V271A | ++ | * |
| V271C | + | ** |
| V271G | ++ | * |
| V271I | + | * |
| V271Q | - | * |
| V271R | + | ** |
| V271V | + | ** |
| L272A | + | ** |
| L272C | + | * |
| L272E | - | * |
| L272G | - | * |
| L272Q | + | * |
| L272S | - | * |
| L272V | ++ | ** |
| L272W | + | * |
| V328G | - | * |
| V328I | +++ | * |
| V328R | ++ | * |
| V328S | ++ | * |
| V328T | + | * |
| V328W | + | * |

| | | |
|---|---|---|
| Note: - represented -50%<e.e.<1 %, + represented 1-30%, ++ represented 30-60%, +++ represented 60-80%, ++++ represented 80-90%, +++++ represented 90-95%, and ++++++ represented greater than 95%. Note: * represented 1-30% of activity, ** represented 30-60%, *** represented 60-70%, **** represented 70-80%, ***** represented 80-90%, ****** represented 90-95%, and ******* represented greater than 95%. | | |

### Embodiment 2

V242W was used as a template, and mutations were continuously performed on 6 sites of L59, F164, L272, V328, T330, A436, and R442, to obtain a total of 40 mutants. Reaction validation was performed on Substrate 1, and a reaction system was as follows: 10 mg of the substrate, 0.1 mg of pyridoxal phosphate, 20 mg of 6 M isopropylamine hydrochloride, and 1 mg of an enzyme were supplemented with 0.1 M phosphate buffer solution pH 7.5 until the total volume was 0.5 mL, and reaction conditions were: 45 °C, 200 rpm, and 16 h. Results were shown in Table 2, e.e. of V242W+F164Q, V242W+L272K, V242W+V328M, V242W+V328I, and V242W+R442T and the like was significantly improved compared to V242W. Due to the higher activity (conversion rate) and selectivity of V242W+F164Q compared to the template V242W, V242W+F164Q was selected as a subsequent template.

**Table 2**

| Mutation site | e.e. % | Activity% |
|---|---|---|
| Template | ++ | *** |
| V242W | +++++ | * |
| V242W+L59Q | ++++ | * |
| V242W+F164C | +++++ | *** |
| V242W+F164Q | ++++++ | *** |
| V242W+F164W | ++++ | * |
| V242W+F164Y | +++++ | ** |
| V242W+L272G | +++++ | ** |
| V242W+L272I | +++++ | * |
| V242W+L272K | ++++++ | * |
| V242W+L272M | +++++ | * |
| V242W+L272P | +++++ | * |
| V242W+L272V | ++++ | ** |
| V242W+L272Y | ++++ | * |
| V242W+V328C | ++++ | * |
| V242W+V328I | ++++++ | * |
| V242W+V328L | ++++++ | * |
| V242W+V328M | ++++++ | * |
| V242W+V328Q | ++++++ | * |
| V242W+V328S | ++++++ | * |
| V242W+V328T | ++++++ | * |
| V242W+V328W | +++++ | * |
| V242W+T330F | +++++ | * |
| V242W+T330I | +++++ | * |
| V242W+T330S | +++++ | * |
| V242W+A436H | +++++ | * |
| V242W+A436K | +++++ | * |
| V242W+A436L | ++++++ | * |
| V242W+A436N | +++++ | * |
| V242W+A436P | ++++++ | * |
| V242W+A436Q | +++++ | * |
| V242W+A436S | +++++ | * |
| V242W+A436Y | +++++ | * |
| V242W+R442A | ++++++ | * |
| V242W+R442C | ++++++ | * |
| V242W+R442F | +++++ | * |
| V242W+R442G | ++++++ | * |
| V242W+R442H | ++++++ | * |
| V242W+R442N | ++++++ | * |
| V242W+R442Q | ++++++ | * |
| V242W+R442S | +++++ | * |
| V242W+R442T | ++++++ | * |

| | | |
|---|---|---|
| Note: - represented -50%<e.e.<1 %, + represented 1-30%, ++ represented 30-60%, +++ represented 60-80%, ++++ represented 80-90%, +++++ represented 90-95%, and ++++++ represented greater than 95%. Note: * represented 1-30% of activity, ** represented 30-60%, *** represented 60-70%, **** represented 70-80%, ***** represented 80-90%, ****** represented 90-95%, and ******* represented greater than 95%. | | |

### Embodiment 3

V242W+F164Q was selected as a template, to construct 17 amino acid sites of V328 and 18 single point mutation sites of R442T. Reaction validation was performed on Substrate 1, and a reaction system was as follows: 10 mg of the substrate, 0.1 mg of pyridoxal phosphate, 20 mg of 6 M isopropylamine hydrochloride, and 1 mg of an enzyme were supplemented with 0.1 M phosphate buffer solution pH 7.5 until the total volume was 0.5 mL, and reaction conditions were: 45 °C, 200 rpm, and 16 h. Results were shown in Table 3, two mutants with significantly improved activity (reflected in the conversion rate) were screened, namely V242W+F164Q+V328I and V242W+F164Q+R442T. V242W+F164Q+V328I was selected as a next template.

**Table 3**

| Mutation site | e.e.% | Conversion rate% |
|---|---|---|
| Template | ++ | *** |
| V242W | +++++ | * |
| V242W+F164Q | ++++++ | *** |
| V242W+F164Q+V328A | ++++ | *** |
| V242W+F164Q+V328C | ++++ | *** |
| V242W+F164Q+V328D | +++++ | *** |
| V242W+F164Q+V328E | ++++++ | *** |
| V242W+F164Q+V328F | +++++ | *** |
| V242W+F164Q+V328G | +++++ | *** |
| V242W+F164Q+V328H | +++++ | *** |
| V242W+F164Q+V328I | ++++++ | **** |
| V242W+F164Q+V328L | ++++ | *** |
| V242W+F164Q+V328M | +++++ | *** |
| V242W+F164Q+V328P | +++++ | *** |
| V242W+F164Q+V328Q | ++++++ | *** |
| V242W+F164Q+V328R | +++++ | *** |
| V242W+F164Q+V328S | +++++ | *** |
| V242W+F164Q+V328W | - | *** |
| V242W+F164Q+V328T | +++++ | *** |
| V242W+F164Q+V328Y | ++++ | *** |
| V242W+F164Q+R442T | ++++++ | **** |

| | | |
|---|---|---|
| Note: - represented -50%<e.e.<1 %, + represented 1-30%, ++ represented 30-60%, +++ represented 60-80%, ++++ represented 80-90%, +++++ represented 90-95%, and ++++++ represented greater than 95%. Note: * represented 1-30% of activity, ** represented 30-60%, *** represented 60-70%, **** represented 70-80%, ***** represented 80-90%, ****** represented 90-95%, and ******* represented greater than 95%. | | |

### Embodiment 4

V242W+F164Q+V328I was selected as a template, to construct 18 single point mutation sites of G2S, T46M, G48D, C185Y, S186N, S194P, T197M, N202D, Y205L, T245A, V252I, S268N, L353F, N359D, R409T, E424K, A436V, and R442. Reaction validation was performed on Substrate 1, a reaction system was as follows: 10 mg of the substrate, 0.1 mg of pyridoxal phosphate, 20 mg of 6 M isopropylamine hydrochloride, and 0.25 mg of an enzyme were supplemented with 0.1 M phosphate buffer solution pH 7.5 until the total volume was 0.5 mL, and reaction conditions were as follows: 45 °C, 200 rpm, and 16 h. The activity of V242W+F164Q+V328I+R442T was increased significantly, so it was used as a next template. Table 4 showed corresponding e.e. and activity results.

**Table 4**

| Mutation site | e.e.% | Conversion rate% |
|---|---|---|
| Template | ++ | ** |
| V242W | +++++ | * |
| V242W+F164Q | ++++++ | ** |
| V242W+F164Q+V328I | ++++++ | *** |
| V242W+F164Q+V328I+G2S | ++++++ | ** |
| V242W+F164Q+V328I+T46M | ++++++ | *** |
| V242W+F164Q+V328I+G48D | ++++++ | *** |
| V242W+F164Q+V328I+C185Y | ++++++ | ** |
| V242W+F164Q+V328I+S186N | ++++++ | *** |
| V242W+F164Q+V328I+S194P | ++++++ | *** |
| V242W+F164Q+V328I+T197M | ++++++ | ** |
| V242W+F164Q+V328I+N202D | ++++++ | ** |
| V242W+F164Q+V328I+Y205L | ++++++ | ** |
| V242W+F164Q+V328I+T245A | ++++++ | ** |
| V242W+F164Q+V328I+V252I | ++++++ | *** |
| V242W+F164Q+V328I+S268N | ++++++ | *** |
| V242W+F164Q+V328I+L353F | ++++++ | *** |
| V242W+F164Q+V328I+N359D | ++++++ | *** |
| V242W+F164Q+V328I+R409T | ++++++ | *** |
| V242W+F164Q+V328I+E424K | ++++++ | *** |
| V242W+F164Q+V328I+A436V | +++++ | ** |
| V242W+F164Q+V328I+R442T | ++++++ | *** |

| | | |
|---|---|---|
| Note: - represented -50%<e.e.<1 %, + represented 1-30%, ++ represented 30-60%, +++ represented 60-80%, ++++ represented 80-90%, +++++ represented 90-95%, and ++++++ represented greater than 95%. Note: * represented 1-30% of activity, ** represented 30-60%, *** represented 60-70%, **** represented 70-80%, ***** represented 80-90%, ****** represented 90-95%, and ******* represented greater than 95%. | | |

### Embodiment 5

V242W+F164Q+V328I+R442T was selected as a template for a single point mutation using primers G48D, S194P, and V252T. Reaction validation was performed on Substrate 1, a reaction system was as follows: 10 mg of the substrate, 0.1 mg of pyridoxal phosphate, 20 mg of 6 M isopropylamine hydrochloride, and 0.25 mg of an enzyme were supplemented with 0.1 M phosphate buffer solution pH 7.5 until the total volume was 0.5 mL, and reaction conditions were as follows: 45 °C, 200 rpm, and 16 h. The activity of V242W+F164Q+V328I+R442T+S194P and V242W+F164Q+V328I+R442T+V252I was the highest, and the protein expression of the mutant was significantly higher in the supernatant according to protein electrophoresis results.

**Table 5**

| Mutation site | e.e.% | Conversion rate% | Protein expression supernatant | Protein expression precipitate |
|---|---|---|---|---|
| Template | ++ | ** | # | #### |
| V242W+F164Q+V328I | ++++++ | *** | #### | ### |
| V242W+F164Q+V328I+R442T | ++++++ | *** | ##### | ### |
| V242W+F164Q+V328I+R442T+G48D | ++++++ | *** | ### | ## |
| V242W+F164Q+V328I+R442T+S194P | ++++++ | **** | ###### | # |
| V242W+F164Q+V328I+R442T+V252I | ++++++ | **** | ###### | # |

| | | | | |
|---|---|---|---|---|
| Note: - represented -50%<e.e.<1 %, + represented 1-30%, ++ represented 30-60%, +++ represented 60-80%, ++++ represented 80-90%, +++++ represented 90-95%, and ++++++ represented greater than 95%. Note: * represented 1-30% of activity, ** represented 30-60%, *** represented 60-70%, **** represented 70-80%, ***** represented 80-90%, ****** represented 90-95%, and ******* represented greater than 95%. # represented 10%-25% of the SDS-PAGE band percentage, ## represented 25%-40% of the SDS-PAGE band percentage, ### represented 40%-55% of the SDS-PAGE band percentage, #### represented 55%-70% of the SDS-PAGE band percentage, ##### represented 70%-85% of the SDS-PAGE band percentage, and ###### represented 85%-90% of the SDS-PAGE band percentage. | | | | |

### Embodiment 6

V242W+F164Q+V328I+R442T+S194P was selected as a template for a single point mutation, with mutation sites V252I and G48D; and V242W+F164Q+V328I+R442T+V252I was selected as a template, with a mutation site G48D. Reaction validation was performed on Substrate 1, a reaction system was as follows: 10 mg of the substrate, 0.1 mg of pyridoxal phosphate, 20 mg of 6 M isopropylamine hydrochloride, and 0.25 mg of an enzyme were supplemented with 0.1 M phosphate buffer solution pH 7.5 until the total volume was 0.5 mL, and reaction conditions were as follows: 45 °C, 200 rpm, and 16 h. Results were shown in Table 6, the activity of the mutant V242W+F164Q+V328I+R442T+V252I+G48D was increased, and the protein expression was significantly higher in the supernatant.

**Table 6**

| Mutation site | e.e.% | Conversion rate % | Protein expression supernatant | Protein expression precipitate |
|---|---|---|---|---|
| Template | ++ | ** | ## | ####### |
| V242W+F164Q+V328I+R442T | ++++++ | *** | ### | ## |
| V242W+F164Q+V328I+R442T+S194P+V252I | ++++++ | **** | ##### | # |
| V242W+F164Q+V328I+R442T+V252I+G48D | ++++++ | ***** | *######* | # |
| V242W+F164Q+V328I+R442T+S194P+G48D | ++++++ | **** | *######* | # |

| | | | | |
|---|---|---|---|---|
| Note: - represented -50%<e.e.<1 %, + represented 1-30%, ++ represented 30-60%, +++ represented 60-80%, ++++ represented 80-90%, +++++ represented 90-95%, and ++++++ represented greater than 95%. Note: * represented 1-30% of activity, ** represented 30-60%, *** represented 60-70%, **** represented 70-80%, ***** represented 80-90%, ****** represented 90-95%, and ******* represented greater than 95%. # represented 10%-25% of the SDS-PAGE band percentage, ## represented 25%-40% of the SDS-PAGE band percentage, ### represented 40%-55% of the SDS-PAGE band percentage, #### represented 55%-70% of the SDS-PAGE band percentage, ##### represented 70%-85% of the SDS-PAGE band percentage, and ###### represented 85%-90% of the SDS-PAGE band percentage. | | | | |

### Embodiment 7

V242W+F164Q+V328I+R442T+S194P+V252I was selected as a template for a single point mutation, with a mutation site G48D. Reaction validation was performed on Substrate 1, a reaction system was as follows: 10 mg of the substrate, 0.1 mg of pyridoxal phosphate, 20 mg of 6 M isopropylamine hydrochloride, and 0.25 mg of an enzyme were supplemented with 0.1 M phosphate buffer solution pH 7.5 until the total volume was 0.5 mL, and reaction conditions were as follows: 45 °C, 200 rpm, and 16 h. Results were shown in Table 7, and the mutant V242W+F164Q+V328I+R442T+S194P+V252I+G48D had the highest activity.

**Table 7**

| Mutation site | Substrate 1 e.e.% | Substrate 1 conversion rate% |
|---|---|---|
| Template | ++ | ** |
| V242W | +++++ | * |
| V242W+F164Q | ++++++ | ** |
| V242W+F164Q+V328I | ++++++ | *** |
| V242W+F164Q+V328I+R442T | ++++++ | *** |
| V242W+F164Q+V328I+R442T+S194P+V252I+G48D | ++++++ | ***** |

| | | |
|---|---|---|
| Note: - represented -50%<e.e.<1 %, + represented 1-30%, ++ represented 30-60%, +++ represented 60-80%, ++++ represented 80-90%, +++++ represented 90-95%, and ++++++ represented greater than 95%. Note: * represented 1-30% of activity, ** represented 30-60%, *** represented 60-70%, **** represented 70-80%, ***** represented 80-90%, ****** represented 90-95%, and ******* represented greater than 95%. | | |

### Embodiment 8

Under the condition of the fixed enzyme amount of 1 mg, the different substrate concentrations of Substrate 1 were verified. Reaction conditions: 0.1 mg of pyridoxal phosphate, different concentrations of 6 M isopropylamine hydrochloride, the total volume of 2 mL, 0.1 M phosphate buffer solution pH 7.5, 45 °C, 200 rpm, and 40 h. Results were shown in Table 8, e.e. might be increased with the increase of the substrate concentration, but the substrate conversion rate might be decreased with the increase of the substrate concentration, and preferably the substrate concentration was 30 mg/mL.

**Table 8**

| Mutation site | Substrate concentration mg/mL | e.e.% | Conversion rate% |
|---|---|---|---|
| Template | 50 | ++ | *** |
| | 30 | ++ | *** |
| | 25 | ++ | *** |
| | 20 | ++ | *** |
| V242W+F164Q+V328I+R442T+S194P+V252I+G48D | 50 | 97.42 | 90.22 |
| | 30 | 96.85 | 92.78 |
| | 25 | 96.47 | 93.16 |
| | 20 | 96.29 | 94.32 |

| | | | |
|---|---|---|---|
| Note: - represented -50%<e.e.<1 %, + represented 1-30%, ++ represented 30-60%, +++ represented 60-80%, ++++ represented 80-90%, +++++ represented 90-95%, and ++++++ represented greater than 95%. Note: * represented 1-30% of activity, ** represented 30-60%, *** represented 60-70%, **** represented 70-80%, ***** represented 80-90%, ****** represented 90-95%, and ******* represented greater than 95%. | | | |

### Embodiment 9

Under the condition of the fixed concentration of Substrate 1, the usage amounts of different enzymes were verified. A reaction system was as follows: 0.5 mg of pyridoxal phosphate, 90 mg of 6 M isopropylamine hydrochloride, 0.1 M phosphate buffer solution pH 7.5, and the total volume of 1.5 mL; and reaction conditions were as follows: 45 °C, 200 rpm, and 40 h. Results were shown in Table 9: as the enzyme amount was decreased, there was a slight increase in e.e., and the mutant activity was not changed much, and preferably the enzyme amount was 1.7 mg/mL.

**Table 9**

| Mutation site | Enzyme amount mg/mL | e.e.% | Conversion rate% |
|---|---|---|---|
| Template | 1.7 | ++ | ** |
| | 3.3 | ++ | *** |
| | 6.6 | ++ | *** |
| V242W+F164Q+V328I+R442T+S194P+V252I+G48D | 1.7 | 97.59 | 93.20 |
| | 3.3 | 96.84 | 94.59 |
| | 6.6 | 95.31 | 93.98 |

| | | | |
|---|---|---|---|
| Note: - represented -50%<e.e.<1 %, + represented 1-30%, ++ represented 30-60%, +++ represented 60-80%, ++++ represented 80-90%, +++++ represented 90-95%, and ++++++ represented greater than 95%. Note: * represented 1-30% of activity, ** represented 30-60%, *** represented 60-70%, **** represented 70-80%, ***** represented 80-90%, ****** represented 90-95%, and ******* represented greater than 95%. | | | |

### Embodiment 10

The reaction temperature was optimized for Substrate 1. A reaction system was as follows: 60 mg of the substrate, 3 mg of an enzyme, 0.6 mg of pyridoxal phosphate, and 120 mg of 6M isopropylamine hydrochloride were supplemented with 0.1 M phosphate buffer solution pH 7.5 until the total volume was 1.8 mL; and reaction conditions were as follows: 45 °C, 200 rpm, and 40 h. Results were shown in Table 10: there was no significant change in e.e. as the temperature was decreased, but the activity was decreased significantly at 20 °C, and preferably it was reacted at 30 °C.

**Table 10**

| Mutant | Temperature | e.e.(%) | Activity % |
|---|---|---|---|
| Template | 20°C | ++ | ** |
| V242W+F164Q+V328I+R442T+S194P+V252I+G48D | | ++++++ | **** |
| Template | 30°C | ++ | *** |
| V242W+F164Q+V328I+R442T+S194P+V252I+G48D | | ++++++ | ****** |
| Template | 37°C | ++ | *** |
| V242W+F164Q+V328I+R442T+S194P+V252I+G48D | | ++++++ | ****** |
| Template | 45°C | ++ | *** |
| V242W+F164Q+V328I+R442T+S194P+V252I+G48D | | ++++++ | ****** |

| | | | |
|---|---|---|---|
| Note: - represented -50%<e.e.<1%, + represented 1-30%, ++ represented 30-60%, +++ represented 60-80%, ++++ represented 80-90%, +++++ represented 90-95%, and ++++++ represented greater than 95%. Note: * represented 1-30% of activity, ** represented 30-60%, *** represented 60-70%, **** represented 70-80%, ***** represented 80-90%, ****** represented 90-95%, and ******* represented greater than 95%. | | | |

### Embodiment 11

A reaction of Substrate 1 was enlarged, and a reaction system was as follows: 1 g of the substrate, 0.05 g of an enzyme, 10 mg of pyridoxal phosphate, and 2 g of 6 M isopropylamine hydrochloride were supplemented with 0.1 M phosphate buffer solution pH 7.5 until the total volume was 30 mL; and reaction conditions were as follows: 30 °C, 200 rpm, and 60 h, and it was reacted in a 250 mL triangular flask. Results were shown in Table 11, and the mutant showed a significant increase in both e.e. and activity compared to a female parent.

**Table 11**

| Mutant | e.e.(%) | Activity(%) |
|---|---|---|
| Template | ++ | **** |
| V242W+F164Q+V328I+R442T+S194P+V252I+G48D | ++++++ | ******* |

| | | |
|---|---|---|
| Note: - represented -50%<e.e.<1 %, + represented 1-30%, ++ represented 30-60%, +++ represented 60-80%, ++++ represented 80-90%, +++++ represented 90-95%, and ++++++ represented greater than 95%. Note: * represented 1-30% of activity, ** represented 30-60%, *** represented 60-70%, **** represented 70-80%, ***** represented 80-90%, ****** represented 90-95%, and ******* represented greater than 95%. | | |

The system was treated after the reaction was completed, and specific steps were as follows: impurities were removed, and the system was transferred to a 50 mL centrifuge tube. Ethyl acetate with a half volume of the system was added and shaken, respectively transferred to two 50 mL centrifuge tubes, and centrifuged at 4000 rpm for 10 min. A supernatant was discarded, a lower system was taken and added with ethyl acetate with a half volume of the system, shaken and centrifuged at 4000 rpm for 10 min. It was repeated once. A lower system was taken and added with ethyl acetate with a half volume of the system, shaken and centrifuged at 4000 rpm for 10 min. It was repeated once. A lower system was taken and transferred to a 250 mL round bottom flask, and weighed. In order to remove the substrate, sodium hydroxide dry powder was added to the system in the round bottom flask, and the addition amount was a half of the weight of the remaining system, it was ensured that the system was below 40°C throughout the entire process. Methyl tert-ether with 0.5 times of the system volume (half of the system volume) was added, transferred to two 50 mL centrifuge tubes, and centrifuged at 4000 rpm for 10 min. A supernatant was added with methyl tert-ether with 0.5 times of the system volume (half of the supernatant volume), and centrifuged at 4000 rpm for 10 min. It was repeated once. The supernatant was transferred to a round bottom flask, and the product yield after rotary distillation was 53%.

### Embodiment 12

Optimized reaction conditions were used to verify Substrate 2, Substrate 3, Substrate 4, and Substrate 5, a reaction system was as follows: 10 mg of the substrate, 0.1 mg of pyridoxal phosphate, 20 mg of 6 M isopropylamine hydrochloride, and 0.5 mg of an enzyme were supplemented with 0.1 M phosphate buffer solution pH 7.5 until the total volume was 0.3 mL, and reaction conditions were as follows: 30°C, 200 rpm, and 60 h. Results were shown in Table 12, and the mutant activity was improved for the different substrates.

**Table 12**

| Mutation site | Substr ate 2 e.e.% | Substr ate 2 activity % | Substr ate 3 e.e.% | Substr ate 3 activity % | Substr ate 4 activity % | Substr ate 5 activity % |
|---|---|---|---|---|---|---|
| Template | + | * | + | * | * | * |
| V242W | ++ | ** | ++ | ** | ** | ** |
| V242W+L59Q | +++ | ** | ++ | ** | ** | ** |
| V242W+F164C | ++ | ** | ++ | ** | ** | ** |
| V242W+F164Q | +++ | ** | +++ | ** | ** | ** |
| V242W+F164W | ++ | ** | ++ | ** | ** | ** |
| V242W+F164Y | ++ | ** | ++ | ** | ** | ** |
| V242W+L272G | ++ | ** | ++ | ** | ** | ** |
| V242W+L272I | ++ | ** | ++ | ** | ** | ** |
| V242W+L272K | ++ | ** | ++ | ** | ** | ** |
| V242W+L272M | ++ | ** | ++ | ** | ** | ** |
| V242W+L272P | ++ | ** | ++ | ** | ** | ** |
| V242W+L272V | ++ | ** | ++ | ** | ** | ** |
| V242W+L272Y | ++ | ** | ++ | ** | ** | ** |
| V242W+V328C | ++ | ** | ++ | ** | ** | ** |
| V242W+V328I | +++ | ** | +++ | ** | ** | ** |
| V242W+V328L | ++ | ** | ++ | ** | ** | ** |
| V242W+V328M | ++ | ** | ++ | ** | ** | ** |
| V242W+V328Q | ++ | ** | ++ | ** | ** | ** |
| V242W+V328S | ++ | ** | ++ | ** | ** | ** |
| V242W+V328T | ++ | ** | ++ | ** | ** | ** |
| V242W+V328W | ++ | ** | ++ | ** | ** | ** |
| V242W+T330F | ++ | ** | ++ | ** | ** | ** |
| V242W+T330I | ++ | ** | ++ | ** | ** | ** |
| V242W+T330S | ++ | ** | ++ | ** | ** | ** |
| V242W+A436H | ++ | ** | ++ | ** | ** | ** |
| V242W+A436K | ++ | ** | ++ | ** | ** | ** |
| V242W+A436L | ++ | ** | ++ | ** | ** | ** |
| V242W+A436N | ++ | ** | ++ | ** | ** | ** |
| V242W+A436P | ++ | ** | ++ | ** | ** | ** |
| V242W+A436Q | +++ | ** | +++ | ** | ** | ** |
| V242W+A436S | ++ | ** | ++ | ** | ** | ** |
| V242W+A436Y | ++ | ** | ++ | ** | ** | ** |
| V242W+R442A | ++ | ** | ++ | ** | ** | ** |
| V242W+R442C | ++ | ** | ++ | ** | ** | ** |
| V242W+R442F | ++ | ** | ++ | ** | ** | ** |
| V242W+R442G | ++ | ** | ++ | ** | ** | ** |
| V242W+R442H | ++ | ** | ++ | ** | ** | ** |
| V242W+R442N | ++ | ** | ++ | ** | ** | ** |
| V242W+R442Q | ++ | ** | ++ | ** | ** | ** |
| V242W+R442S | ++ | ** | ++ | ** | ** | ** |
| V242W+R442T | +++ | ** | ++ | ** | ** | ** |
| V242W+F164Q+V328A | ++ | ** | +++ | ** | ** | ** |
| V242W+F164Q+V328C | +++ | ** | +++ | ** | ** | ** |
| V242W+F164Q+V328D | ++ | ** | +++ | ** | ** | ** |
| V242W+F164Q+V328E | ++ | ** | +++ | ** | ** | ** |
| V242W+F164Q+V328F | +++ | ** | +++ | ** | ** | ** |
| V242W+F164Q+V328G | ++ | ** | +++ | ** | ** | ** |
| V242W+F164Q+V328H | +++ | ** | +++ | ** | ** | ** |
| V242W+F164Q+V328I | +++ | ** | +++ | ** | ** | ** |
| V242W+F164Q+V328L | +++ | ** | +++ | ** | ** | ** |
| V242W+F164Q+V328M | ++ | ** | +++ | ** | ** | ** |
| V242W+F164Q+V328P | +++ | ** | +++ | ** | ** | ** |
| V242W+F164Q+V328Q | +++ | ** | +++ | ** | ** | ** |
| V242W+F164Q+V328R | +++ | ** | +++ | ** | ** | ** |
| V242W+F164Q+V328S | ++ | ** | +++ | ** | ** | ** |
| V242W+F164Q+V328W | ++ | ** | +++ | ** | ** | ** |
| V242W+F164Q+V328T | +++ | ** | +++ | ** | ** | ** |
| V242W+F164Q+V328Y | +++ | ** | +++ | ** | ** | ** |
| V242W+F164Q+R442T | +++ | ** | +++ | ** | ** | ** |
| V242W+F164Q+V328I+G2S | ++++ | *** | +++ | ** | *** | *** |
| V242W+F164Q+V328I+T46M | +++ | *** | +++ | ** | *** | *** |
| V242W+F164Q+V328I+G48D | ++++ | *** | ++++ | ** | *** | *** |
| V242W+F164Q+V328I+C185Y | ++++ | *** | +++ | ** | *** | *** |
| V242W+F164Q+V328I+S186N | +++ | *** | +++ | ** | *** | *** |
| V242W+F164Q+V328I+S194P | +++ | *** | ++++ | ** | *** | *** |
| V242W+F164Q+V328I+T197M | +++ | *** | +++ | ** | *** | *** |
| V242W+F164Q+V328I+N202D | +++ | *** | +++ | ** | *** | *** |
| V242W+F164Q+V328I+Y205L | +++ | *** | +++ | ** | *** | *** |
| V242W+F164Q+V328I+T245A | +++ | *** | +++ | ** | *** | *** |
| V242W+F164Q+V328I+V2521 | ++++ | *** | ++++ | ** | *** | *** |
| V242W+F164Q+V328I+S268N | +++ | *** | +++ | ** | *** | *** |
| V242W+F164Q+V328I+L353F | +++ | *** | +++ | ** | *** | *** |
| V242W+F164Q+V328I+N359D | +++ | *** | +++ | ** | *** | *** |
| V242W+F164Q+V328I+R409T | +++ | *** | +++ | ** | *** | *** |
| V242W+F164Q+V328I+E424K | ++++ | *** | +++ | ** | *** | *** |
| V242W+F164Q+V328I+A436V | ++++ | *** | ++++ | ** | *** | *** |
| V242W+F164Q+V328I+R442T | ++++ | **** | ++++ | ** | **** | **** |
| V242W+F164Q+V328I+R442T+G48D | ++++ | ***** | ++++ | *** | **** | **** |
| V242W+F164Q+V328I+R442T+S194P | ++++ | ***** | ++++ | *** | **** | **** |
| V242W+F164Q+V328I+R442T+V252I | ++++ | ***** | ++++ | *** | **** | **** |
| V242W+F164Q+V328I+R442T+V252I+ G48D | +++++ | ***** | ++++ | **** | ***** | ***** |
| V242W+F164Q+V328I+R442T+S194P+ G48D | +++++ | ***** | ++++ | **** | ***** | ***** |
| V242W+F164Q+V328I+R442T+S194P+ V252I+G48D | +++++ | ****** | +++++ | ***** | ****** | ****** |

| | | | | | | |
|---|---|---|---|---|---|---|
| Note: - represented -50%<e.e.<1 %, + represented 1-30%, ++ represented 30-60%, +++ represented 60-80%, ++++ represented 80-90%, +++++ represented 90-95%, and ++++++ represented greater than 95%. Note: * represented 1-30% of activity, ** represented 30-60%, *** represented 60-70%, **** represented 70-80%, ***** represented 80-90%, ****** represented 90-95%, and ******* represented greater than 95%. | | | | | | |

In addition, by computer simulation analysis of the three-dimensional structure of the transaminase using software, it is found that most of the mutated sites are located near the active center. After mutations, it is possible to enhance the binding between the substrate and enzyme, thereby the selectivity and catalytic efficiency are improved.

The above are only preferred embodiments of the present disclosure and are not intended to limit the present disclosure. For those skilled in the art, the present disclosure may have various modifications and variations. Any modifications, equivalent replacements, improvements and the like made within the spirit and principles of the present disclosure shall be contained within the scope of protection of the present disclosure.

## Claims

1. A transaminase mutant, wherein the transaminase mutant has an amino acid sequence obtained by the mutation of the amino acid sequence shown in SEQ ID NO: 1, and the mutation comprises V242W, or an amino acid sequence has the mutation and has more than 90% homology with SEQ ID NO: 1and has transaminase activity.

2. The transaminase mutant according to claim 1, wherein the mutation comprises any one of the following mutation site combinations: V242W+L59Q , V242W+F164C, V242W+F164Q, V242W+F164W , V242W+F164Y , V242W+L272G, V242W+L272I, V242W+L272K, V242W+L272M, V242W+L272P, V242W+L272V, V242W+L272Y, V242W+V328C, V242W+V328I, V242W+V328L, V242W+V328M, V242W+V328Q, V242W+V328S, V242W+V328T, V242W+V328W, V242W+T330F, V242W+T330I, V242W+T330S, V242W+A436H, V242W+A436K, V242W+A436L, V242W+A436N, V242W+A436P, V242W+A436Q, V242W+A436S, V242W+A436Y, V242W+R442A, V242W+R442C, V242W+R442F, V242W+R442G, V242W+R442H, V242W+R442N, V242W+R442Q, V242W+R442S, V242W+R442T, V242W+F164Q+V328A, V242W+F164Q+V328C, V242W+F164Q+V328D, V242W+F164Q+V328E, V242W+F164Q+V328F, V242W+F164Q+V328G, V242W+F164Q+V328H, V242W+F164Q+V328I, V242W+F164Q+V328L, V242W+F164Q+V328M, V242W+F164Q+V328P, V242W+F164Q+V328Q, V242W+F164Q+V328R, V242W+F164Q+V328S, V242W+F164Q+V328W, V242W+F164Q+V328T, V242W+F164Q+V328Y, V242W+F164Q+R442T, V242W+F164Q+V328I+G2S, V242W+F164Q+V328I+T46M, V242W+F164Q+V328I+G48D, V242W+F164Q+V328I+C185Y, V242W+F164Q+V328I+S186N, V242W+F164Q+V328I+S194P, V242W+F164Q+V328I+T197M, V242W+F164Q+V328I+N202D, V242W+F164Q+V328I+Y205L , V242W+F164Q+V328I+T245A, V242W+F164Q+V328I+V252I, V242W+F164Q+V328I+S268N, V242W+F164Q+V328I+L353F, V242W+F164Q+V328I+N359D, V242W+F164Q+V328I+R409T, V242W+F164Q+V328I+E424K, V242W+F164Q+V328I+A436V, V242W+F164Q+V328I+R442T, V242W+F164Q+V328I+R442T+G48D, V242W+F164Q+V328I+R442T+S194P, V242W+F164Q+V328I+R442T+V252I, V242W+F164Q+V328I+R442T+S194P+V252I, V242W+F164Q+V328I+R442T+V252I+G48D, V242W+F164Q+V328I+R442T+S194P+G48D or V242W+F164Q+V328I+R442T+S194P+V252I+G48D.

3. A DNA molecule, wherein the DNA molecule encodes the transaminase mutant of claim 1 or 2.

4. A recombinant plasmid, wherein the recombinant plasmid contains the DNA molecule of claim 3.

5. The recombinant plasmid according to claim 4, wherein the recombinant plasmid is pET-22a (+), pET-22b (+), pET-3a (+), pET-3d (+), pET-11a (+), pET-12a (+), pET-14b (+), pET-15b (+), pET-16b (+), pET-17b (+), pET-19b (+), pET-20b (+), pET-21a (+), pET-23a (+), pET-23b (+), pET-24a (+), pET-25b (+), pET-26b (+), pET-27b (+), pET-28a (+), pET-29a (+), pET-30a (+), pET-31b (+), pET-32a (+), pET-35b (+), pET-38b (+), pET-39b (+), pET-40b (+), pET-41a (+), pET-41b (+), pET-42a (+), pET-43a (+), pET-43b (+), pET-44a (+), pET-49b (+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-18, pUC-18 or pUC-19.

6. A host cell, wherein the host cell contains the recombinant plasmid of claim 4 or 5.

7. The host cell according to claim 6, wherein the host cell comprises a prokaryotic cell or a eukaryotic cell.

8. The host cell according to claim 7, the prokaryotic cell is a BL21-DE3 cell or an *Escherichia coli* Rosetta-DE3 cell; and the eukaryotic cell is a yeast cell.

9. A method for producing a chiral amine, comprising a step of a performing catalytic transamination on a ketone compound and an amino donor using a transaminase, wherein the transaminase is the transaminase mutant of claim 1 or 2.

10. The method according to claim 9, wherein the ketone compound is 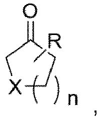 n=0, 1, 2 or 3; X=C, N, O or S; R=H, F, Cl, Br, CH₃ or CH₂CH₃.

11. The method according to claim 9, wherein the ketone compound is

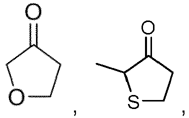

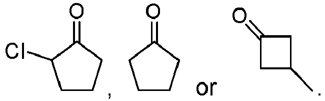

12. The method according to claim 9, wherein the amino donor is isopropamide or alanine.

13. The method according to claim 12, wherein the amino donor is isopropamide.

14. The method according to claim 9, wherein in a reaction system for performing the catalytic transamination on the ketone compound and the amino donor using the transaminase, the transaminase has an amount of 1.5-6.6 mg/mL.

15. The method according to claim 14, wherein in a reaction system for performing the catalytic transamination on the ketone compound and the amino donor using the transaminase, the transaminase has an amount of 1.7 mg/mL.

16. The method according to claim 14, wherein in the reaction system for performing the catalytic transamination on the ketone compound and the amino donor using the transaminase, a temperature is 20 °C-45 °C.

17. The method according to claim 16, wherein in the reaction system for performing the catalytic transamination on the ketone compound and the amino donor using the transaminase, a temperature is 30 °C.
